# EUROPEAN PATENT APPLICATION

(11) **EP 3 950 953 A1**
(43) Date of publication of application: **09.02.2022**
(21) Application number: 20784667.6
(22) Date of filing: 27.03.2020
(51) Int. Cl.: C12Q 1/02, C12Q 1/6851, G01N 33/15, G01N 33/50

(54) **SCREENING METHOD AND TOXICITY EVALUATION METHOD**

(30) Priority: 29.03.2019 JP 2019066625
(71) Applicant: Public University Corporation Yokohama City University, Yokohama-shi, Kanagawa 236-0027 (JP); Takeda Pharmaceutical Company Limited, Osaka-shi, Osaka 541-0045 (JP)
(72) Inventor: TAKEBE, Takanori, Tokyo 113-8510 (JP); SAIKI, Norikazu, Tokyo 113-8510 (JP); NIO, Yasunori, Fujisawa-shi, Kanagawa 251-0012 (JP); KAWAKAMI, Eri, Fujisawa-shi, Kanagawa 251-0012 (JP)
(74) Representative: Jones, Nicholas Andrew
(86) International application number: PCT/JP2020/015255
(87) International publication number: WO 2020/204149

(57) **Abstract**

A method for screening for a therapeutic drug for a disease involving excessive activation of a complement, using a cytotoxicity marker associated with the complement as an index, including (1a) a step of adding a complement to a cell produced from a stem cell to cause the cell to form a cytotoxicity marker associated with the complement, and (2a) a step of adding a therapeutic drug candidate, and selecting a substance that decreases the amount of the cytotoxicity marker is provided by the present invention.

SEQUENCE LISTING

<110>  Public University Corporation Yokohama City University
       Takeda Pharmaceutical Company Limited

<120>  Screening method and toxicity evaluation method

<130>  093019

<150>  JP 2019-066625
<151>  2019-03-29

<160>  6

<170>  PatentIn version 3.5

<210>  1
<211>  18
<212>  DNA
<213>  Artificial Sequence

<220>
<223>  PCR Primer

<400>  1
tcagaccctg agccacct                                                    18


<210>  2
<211>  20
<212>  DNA
<213>  Artificial Sequence

<220>
<223>  PCR Primer

<400>  2
agcaacggac agatgtgtga                                                  20


<210>  3
<211>  23
<212>  DNA
<213>  Artificial Sequence

<220>
<223>  PCR Primer

<400>  3
tccttgtaag tggcttcttg aac                                              23


<210>  4
<211>  27
<212>  DNA
<213>  Artificial Sequence

<220>
<223>  PCR Primer

<400>  4
tgtactgcag agataagttt agctgac                                          27

<210>  5
<211>  20
<212>  DNA
<213>  Artificial Sequence

<220>
<223>  PCR Primer

<400>  5
cttcccttca tcccgaagtt                                                    20


<210>  6
<211>  19
<212>  DNA
<213>  Artificial Sequence

<220>
<223>  PCR Primer

<400>  6
aatgttgcca agctgctga                                                     19

## Description

### [Technical Field]

The present invention relates to a method for screening for a therapeutic drug for a disease involving excessive activation of a complement, using a cytotoxicity marker associated with the complement as an index. The present invention also relates to a method for evaluating cytotoxicity of a test substance caused by excessive activation of a complement, using a cytotoxicity marker associated with the complement as an index.

### [Background of the Invention]

Complement is not only an important effector of innate immunity, but also involved in the acquired immune response, inflammation, blood coagulation, and promotion of tumor progression. As an exogenous substance elimination mechanism by activation of complement, opsonification, anaphylatoxin (C5a,C3a) production, and formation of the final product, membrane attack complex of complements (MAC), can be mentioned. While the activation of complement functions as a biological defense, it has the risk of exhibiting biological cytotoxicity in pathological conditions. For example, in the kidney, complement deposition in renal tissue causes membranoproliferative glomerulonephritis (MPGN), poststreptococcal acute glomerulonephritis (PSAGN), and lupus nephritis. In addition, activation of complement forms a membrane attack complex (MAC), and the formation of MAC creates structural pores in the cell membrane. As a result, movement of osmotic pressure fluid and influx of cation occur, which in turn causes cell death. Since abnormal activation of complement causes various diseases as described above, screening for a therapeutic agent for diseases involving excessive activation of complement has been performed using complement activation as an index and taking note of hemolytic action. However, these conventional methods do not take into consideration cytotoxicity such as vascular injury and the like that actually occur due to the activation of complement.

Non-patent document 1 has reported that deposition of MAC also occurs in various cancer tissues, alternative pathway of the complement system in osteosarcoma cells can be activated using normal human serum (NHS), and production of growth factors that promote angiogenesis of human endothelial cells can be enhanced. In this document, however, it has also been reported that no difference is found in the degree of generation of MAC between "normal human serum" and "human serum inactivated by heat treatment" in HUVEC (human umbilical vein endothelial cell) and the like other than human osteosarcoma epithelial cell U2-OS.

### [prior art document]

### [non-patent document]

[non-patent document 1] Jeon H. et al., Sci Rep (2018), 8(1): 5415

### [Summary of Invention]

### [Technical Problem]

The problem of the present invention is to provide a method for screening for a therapeutic drug for a disease involving excessive activation of a complement, using a cytotoxicity marker associated with the complement as an index, and a kit used for the screening.

### [Solution to Problem]

The present inventors have contacted vascular endothelial cells induced to differentiate from induced pluripotent stem cell with normal human serum (NHS) containing complement or NHS heat-treated to inactivate complement, and found that a significant difference in the amount of membrane attack complex (MAC) deposition occurs depending on the presence or absence of complement activity. This finding was surprisingly different from the results when human umbilical vein endothelial cells were used as disclosed in non-patent document 1. Moreover, they have found that the amount of MAC deposition using a complement having activity decreases in a dose-dependent manner by the administration of Eculizumab which is an antibody against the complement component C5 at the end of the complement activation cascade, and is also used as a therapeutic agent for atypical hemolytic uremic syndrome (aHus). Based on these findings, the present inventors have induced differentiation of plural cells other than vascular endothelial cell from pluripotent stem cell in the same manner as with the vascular endothelial cell, and performed MAC deposition experiment using the cells and complement. As a result, they have found that a complement having activity causes MAC deposition regardless of cell type, and the amount of MAC deposition decreases in a dose-dependent manner by the administration of Eculizumab. The present inventors have further studied based on these findings and completed the present invention.

To solve the above-mentioned problems, the present invention provides the following [1] - [6].
[1] A method for screening for a therapeutic drug for a disease involving excessive activation of a complement, using a cytotoxicity marker associated with the complement as an index, comprising
   (1a) a step of adding a complement to a cell produced from a stem cell to cause the cell to form a cytotoxicity marker associated with the complement, and
   (2a) a step of adding a therapeutic drug candidate, and selecting a substance that decreases the amount of the cytotoxicity marker.
[2] The method of the aforementioned [1], further comprising adding a complement activation factor in step (1a).
[3] A method for evaluating cytotoxicity of a test substance caused by excessive activation of a complement, using a cytotoxicity marker associated with the complement as an index, comprising
   (1b) a step of adding a test substance to a cell produced from a stem cell,
   (2b) a step of measuring the production amount of the cytotoxicity marker associated with the complement, and
   (3b) a step of evaluating cytotoxicity caused by excessive activation of the complement from the production amount of the cytotoxicity marker.
[4] The method of the aforementioned [1] or [3], wherein the cell produced from a stem cell is a vascular endothelial cell, a hepatic sinusoidal endothelial cell, a nerve cell, an oligodendrocyte, a hepatocyte or a retinal pigment epithelial cell.
[5] A kit for screening for a therapeutic drug for a disease involving excessive activation of a complement, comprising
   (i-a) a cell produced from a stem cell,
   (ii-a) a complement or a complement source, and
   (iii-a) a reagent that detects a cytotoxicity marker associated with the complement.
[6] A kit for evaluating cytotoxicity caused by excessive activation of a complement
   (i-b) a cell produced from a stem cell, and
   (ii-b) a reagent that detects a cytotoxicity marker associated with the complement.

### [Advantageous Effects of Invention]

According to the present invention, a method for screening for a therapeutic drug for a disease involving excessive activation of a complement, using a cytotoxicity marker associated with the complement as an index is provided. It was confirmed that known therapeutic drugs for the aforementioned disease decrease formation of the aforementioned cytotoxicity marker. Using the cytotoxicity marker as an index, therefore, more reliable screening reflecting the influence of blood vessel injury due to a complement actually generated in vivo can be expected.

### [Brief Description of Drawings]

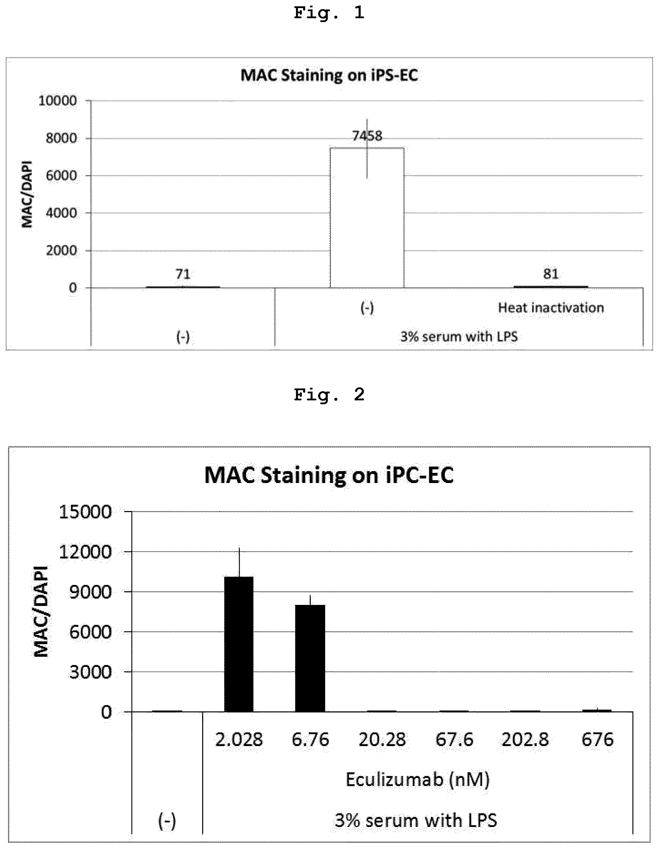

Fig. 1 shows MAC formation after culturing vascular endothelial cell produced from human pluripotent stem cell in a medium containing a complement source (human serum and organoid culture supernatant). In the Figure, - shows the results of an experiment without using a complement source, and 3% serum with LPS shows the results of an experiment using 3% human serum and organoid culture supernatant after LPS treatment as a complement source. Heat inactivation shows the results with a complement source inactivated by allowing it to stand at 55°C for 30 min to 60 min. The MAC immunostaining results were quantified, and the results corrected by nuclear staining (DAPI) quantified in the same manner were used as the results.
Fig. 2 shows formation of MAC after culturing vascular endothelial cell produced from human pluripotent stem cell in a medium containing a complement source (human serum and organoid culture supernatant). In the Figure, - shows the results of an experiment without using a complement source, and Eculizumab and 3% serum with LPS shows the results of an experiment of MAC formation by adding various concentrations of C5 antibody to (3% human serum and organoid culture supernatant after LPS treatment). The MAC immunostaining results were quantified, and the results corrected by nuclear staining (DAPI) quantified in the same manner were used as the results.

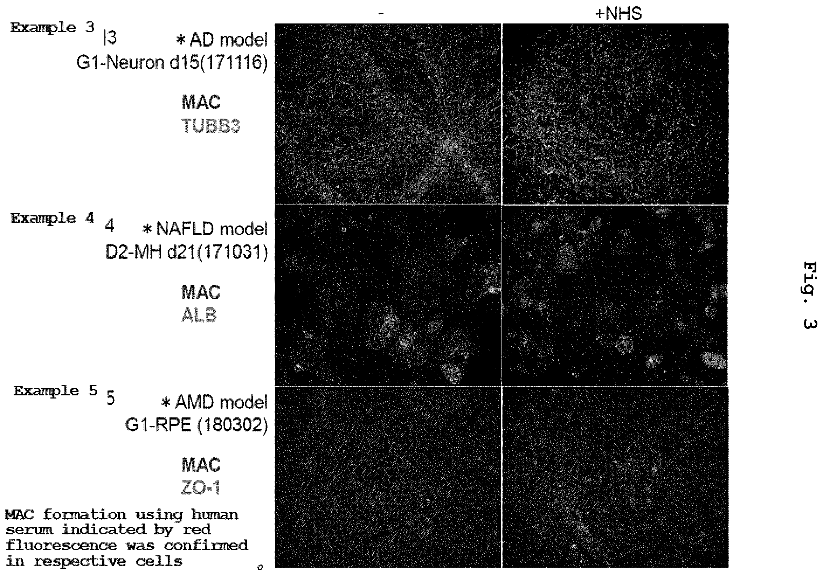

Fig. 3 shows immunostaining diagrams for detection of MAC formation after culturing respective cells (nerve cell, hepatocyte and retinal pigment epithelial cell) produced from human pluripotent stem cell in a medium containing a complement source (human serum and organoid culture supernatant). In the Figure, - shows the results of an experiment without using a complement source, and +NHS shows the results of an experiment using a complement source.

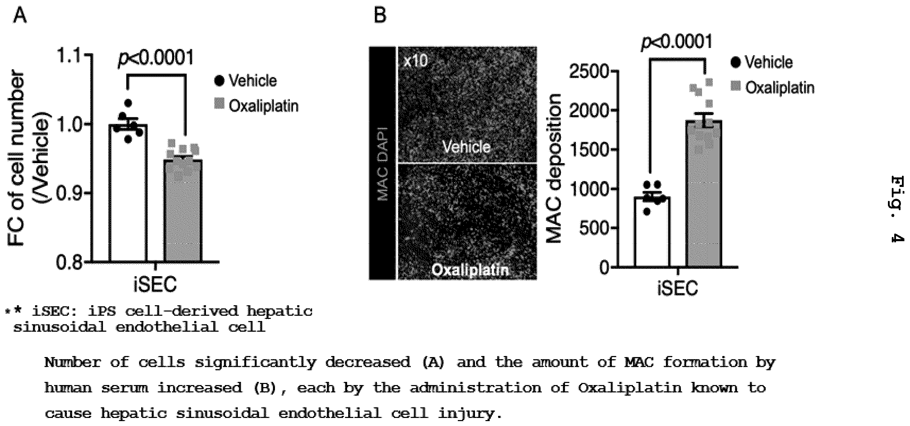

Fig. 4 shows that the number of cells significantly decreased (A) and the amount of MAC formation by human serum increased (B), each by the administration of Oxaliplatin known to cause hepatic sinusoidal endothelial cell injury.

### [Detailed Description of the Invention]

### -1. A method for screening for a therapeutic drug for a disease involving excessive activation of a complement -

The present invention provides a method for screening for a therapeutic drug for a disease involving excessive activation of a complement, using a cytotoxicity marker associated with the complement (hereinafter sometimes to be simply referred to as a "cytotoxicity marker") as an index (hereinafter sometimes to be referred to as "the screening method of the present invention"). The screening method of the present invention includes
(1a) a step of adding a complement to a cell produced from a stem cell to cause the cell to form a cytotoxicity marker, and
(2a) a step of adding a therapeutic drug candidate, and selecting a substance that decreases the amount of the cytotoxicity marker.

In the present specification, "containing (comprise(s) or comprising)" indicates inclusion of the element following the phrase, but means being not limited thereto. Therefore, it suggests inclusion of the element following the phrase, but does not suggest exclusion of any other elements.

Examples of the disease involving excessive activation of a complement include atypical hemolytic uremic syndrome (aHUS), ALZHEIMER, multiple sclerosis (MS), nonalcoholic steatohepatitis (NASH), age-related macular degeneration (AMD), paroximal nocturnal hemogrobinuria (PNH), Shiga toxin-producing Escherichia coli-hemolytic uremic syndrome (STEC-HUS), acute humoral rejection (AHR) (acute antibody-mediated rejection (AMR)), myasthenia gravis, optic nerve myelitis, membranoproliferative glomerulonephritis (MPGN), dense deposit disease (DDD), cold agglutinin disease, catastrophic antiphospholipid syndrome (CAPS) and the like.

In the present specification, examples of the cytotoxicity marker include MAC, lactate dehydrogenase (LDH), cell-mediated cytotoxicity (BrdU uptake), intracellular ATP, DNA fragmentation, Caspase-3/7, 8, 9 reducing ability (NADH and the like) and the like. In the present specification, that the cytotoxicity marker can be formed means that the amount of the cytotoxicity marker (e.g., MAC) formed (deposited) increases as compared with cells before contact with the complement, cells contacted with inactivated complement, or cells without contact with the complement.

As shown in the Examples described below, in multiple types of cells prepared from stem cells, it was demonstrated that MAC deposition occurs due to a complement having activity, and the amount of MAC deposition decreases in a dose-dependent manner by administration of eculizumab. This result was different from the result of human umbilical vein endothelial cells disclosed in non-patent document 1. The reason for such difference may be that the cell population used in non-patent document 1 was separated from the living body, and therefore, was a nonuniform cell population containing a mixture of cells with a plurality of properties depending on the isolation method, and the like. The above-mentioned problem of nonuniform cell population can be avoided by preparing the cells to be used from stem cells. Thus, a cell type produced from a stem cell can be applied to the screening method of the present invention without particular limitations. Examples of such cell type include differentiated cells of nerve cell, oligodendrocyte, erythrocyte, mononuclear cell (e.g., lymphocyte (NK cell, B cell, T cell, monocyte, dendritic cell, etc.)), granulocyte (e.g., eosinophil, neutrophil, basophil), megakaryocyte, epithelial cell (e.g., retinal pigment epithelial cell, etc.), endothelial cell (e.g., vascular endothelial cell, hepatic sinusoidal endothelial cell, etc.), muscle cell, fibroblast (e.g., skin cell, etc.), hair cell, hepatocyte, gastric mucosa, enterocyte, splenocyte, pancreatic cell (e.g., pancreatic exocrine cell, etc.), brain cell, lung cell, kidney cell, adipocyte and the like, immature cells of these, and the like. Among these, vascular endothelial cell, nerve cell, oligodendrocyte, hepatocyte, retinal pigment epithelial cell and immature cells of these are preferable.

As a method for producing (inducing differentiation) the aforementioned cells from stem cells, a method known per se can be appropriately selected according to the type of the desired cell. Examples of the method for producing vascular endothelial cell include the method described in Yamashita J. et al., Nature, 408(6808):92-96 (2000), the method described in Narazaki G. et al., Circulation. 29; 118(5):498-506 (2008), and the like, examples of the method for producing nerve cell include the method described in Yan Y. et al., Stem Cells Trans Med, 2:862-870 (2013), the method described in Kondo T., et al., Cell Stem Cell, 12:487-496 (2013), the method described in WO2015/020234, the method described in Doi D., et al., Stem Cell Reports, 2(3):337-50 (2014), and the like, examples of the method for producing oligodendrocyte include the method described in Kawabata S. et al., Stem Cell Reports, 6(1):1-8 (2016), and the like, examples of the method for producing hepatocyte include the method described in Cai J. et al., Hepatology, 45:1229-1239 (2007), the method described in Cai J. et al., Hepatology, 45:1229-1239 (2007), and the like, examples of the method for producing retinal pigment epithelial cell include the method described in Yan Y. et al., Stem Cells Trans Med, 2:862-870 (2013), and the method described in WO 2015053375, examples of the method for producing hematopoietic progenitor cell include the method described in WO2013/075222, the method described in WO2016/076415, the method described in Liu S. et al., Cytotherapy, 17:344-358 (2015), and the like, examples of the method for producing erythrocyte or erythrocyte progenitor cell include the method described in Miharada K. et al., Nat. Biotechnol., 24:1255-1256(2006), the method described in Kurita R., et al., PLoS One, 8:e59890 (2013), and the like, examples of the method for producing megakaryocyte or platelet include the method described in Yamamizu K. et al., J. Cell Biol., 189:325-338 (2010), the method described in Laflamme M. et al., Nat. Biotechnol., 25:1015-1024 (2007), and the like, examples of the method for producing T cell include the method described in WO2016/076415, the method described in WO2017/221975, and the like, examples of the method for producing skeletal muscle cell include the method described in WO2013/073246, the method described in Uchimura T. et al,. Stem Cell Research, 25:98-106 (2017), the method described in Shoji E. et al., Science Reports, 5:12831 (2015), and the like, and examples of the method for producing cardiomyocyte include the method described in Shimoji K., et al., Cell Stem Cell, 6:227-237 (2010), and the like.

As a specific explanation, to produce vascular endothelial cells from stem cells, for example, a stem cell is cultured in a medium containing a serum replacement (e.g., B-27), BMP4 and GSK-3 inhibitor (e.g., CHIR99021) to induce same into a mesoderm cell, and the cell is cultured in a medium containing VEGF and Folskolin, whereby a vascular endothelial cell can be produced. Also, to produce hepatocyte from a stem cell, a stem cell is cultured in a medium containing a Wnt protein (e.g., Wnt3a) and activin A to induce same into an endodermal cell, and the cell is cultured in a medium containing a serum replacement (B-27) and FGF2, whereby a hepatocyte can be produced.

Examples of the aforementioned "stem cell" include a pluripotent stem cell. In the present specification, the "pluripotent stem cell" refers to a stem cell that can differentiate into tissues and cells with various different morphologies and functions of the living body and is capable of differentiating into cells of any lineage of trigerm (endoderm, mesoderm, ectoderm). The pluripotent stem cell is not particularly limited, and examples thereof include induced pluripotent stem cell (sometimes to be referred to as "iPS cell" in the present specification), embryonic stem cell (ES cell), embryonic stem cell derived from clone embryo obtained by nucleus transplantation (nuclear transfer Embryonic stem cell: ntES cell), pluripotent germ stem cell, embryonic germ cell (EG cell) and the like. In the present specification, the "multipotent stem cell" refers to a stem cell capable of differentiating into cells of a plural restricted number of lineages. Examples of the "multipotent stem cell" include pulp stem cell, mouth cavity mucosa-derived stem cell, hair follicle stem cell, somatic stem cells derived from culture fibroblast and myeloid stem cell, and the like. Preferred pluripotent stem cells are ES cell and iPS cell. When the above-mentioned pluripotent stem cell is ES cell or any cell derived from human embryo, it may be a cell produced by destroying an embryo or without destroying an embryo. Preferred is a cell produced without destroying an embryo. The above-mentioned stem cell is preferable derived from a mammal (e.g., mouse, rat, hamster, guinea pig, dog, monkey, orangutan, chimpanzee, human), more preferably derived from a human.

The "induced pluripotent stem cell" refers to a cell obtained by reprogramming by introducing a specific factor (nuclear reprogramming factor) into a mammalian somatic cell or undifferentiated stem cell. At present, there are various types of "induced pluripotent stem cells". In addition to the iPS cell established by Yamanaka et al. by introducing four factors of Oct3/4, Sox2, Klf4, and c-Myc into mouse fibroblasts (Takahashi K, Yamanaka S., Cell, (2006) 126: 663-676), human cell-derived iPS cells established by introducing the same four factors into human fibroblasts (Takahashi K, Yamanaka S., et al. Cell, (2007) 131: 861-872.), Nanog-iPS cell established by selecting Nanog expression as an index after introducing the above-mentioned four factors (Okita, K., Ichisaka, T., and Yamanaka, S. (2007). Nature 448, 313-317.), iPS cell prepared by a method that does not contain c-Myc (Nakagawa M, Yamanaka S., et al. Nature Biotechnology, (2008) 26, 101-106), and iPS cell established by introducing six factors by a virus-free method (Okita K et al. Nat. Methods 2011 May; 8(5):409-12, Okita K et al. Stem Cells. 31(3):458-66.) can also be used. In addition, induced pluripotent stem cell established by introducing four factors of OCT3/4, SOX2, NANOG, and LIN28 produced by Thomson et al. (Yu J., Thomson JA. et al., Science (2007) 318: 1917-1920.), induced pluripotent stem cell produced by Daley et al. (Park IH, Daley GQ. et al., Nature (2007) 451: 141-146), induced pluripotent stem cell produced by Sakurada et al. (JP-A-2008-307007), and the like can also be used.

In addition, any of the induced pluripotent stem cells known in the art which are described in all published papers (e.g., Shi Y., Ding S., et al., Cell Stem Cell, (2008) Vol3, Issue 5, 568-574; , Kim JB., Scholer HR., et al., Nature, (2008) 454, 646-650; Huangfu D., Melton, DA., et al., Nature Biotechnology, (2008) 26, No 7, 795-797) or patents (e.g., JP-A-2008-307007, JP-A-2008-283972, US2008-2336610, US2009-047263, WO2007-069666, WO2008-118220, WO2008-124133, WO2008-151058, WO2009-006930, WO2009-006997, WO2009-007852) can be used. As the induced pluripotent cell line, various iPS cell lines established by NIH, RIKEN, Kyoto University, and the like can be used. For example, in the case of human iPS cell line, HiPS-RIKEN-1A strain, HiPS-RIKEN-2A strain, HiPS-RIKEN-12A strain, Nips-B2 strain of RIKEN, 253G1 strain, 201B7 strain, 409B2 strain, 454E2 strain, 606A1 strain, 610B1 strain, 648A1 strain of Kyoto University, and the like can be mentioned.

ES cell is a pluripotent and self-replicating stem cell established from the inner cell mass of early embryo (e.g., blastocyst) of a mammal such as human, mice, or the like. ES cell was discovered in mouse in 1981 (M.J. Evans and M.H. Kaufman (1981), Nature 292:154-156), after which ES cell line was also established in primates such as human, monkey and the like (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cell can be established by removing the inner cell mass from the blastocyst of a fertilized egg of the target animal and culturing the inner cell mass on a fibroblast feeder. Methods for establishing and maintaining human and monkey ES cells are described in, for example, USP5,843,780; Thomson JA, et al. (1995), Proc Natl. Acad. Sci. USA. 92:7844-7848; Thomson JA, et al. (1998), Science. 282:1145-1147; Suemori H. et al. (2006), Biochem. Biophys. Res. Commun., 345:926-932; Ueno M. et al. (2006), Proc. Natl. Acad. Sci. USA, 103:9554-9559; Suemori H. et al. (2001), Dev. Dyn., 222:273-279; Kawasaki H. et al. (2002), Proc. Natl. Acad. Sci. USA, 99:1580-1585; Klimanskaya I. et al. (2006), Nature. 444:481-485 and the like. Alternatively, ES cell can be established using only single blastomere of an embryo in the cleavage stage before the blastocyst stage (Chung Y. et al. (2008), Cell Stem Cell 2: 113-117), or can also be established using embryo that has stopped developing (Zhang X. et al. (2006), Stem Cells 24: 2669-2676.). As for "ES cell", various mouse ES cell lines established by inGenious targeting laboratory, RIKEN, and the like can be used for mouse ES cells. For human ES cell, various human ES cell lines established by the University of Wisconsin, NIH, RIKEN, Kyoto University, the National Center for Child Health and Development, Cellartis, and the like can be used. For example, as human ES cell lines, CHB-1 - CHB-12 strains, RUES1 strain, RUES2 strain, HUES1 - HUES28 strains and the like sold by ESI Bio, H1 strain, H9 strain and the like sold by WiCell Research, KhES-1 strain, KhES-2 strain, KhES-3 strain, KhES-4 strain, KhES-5 strain, SSES1 strain, SSES2 strain, SSES3 strain sold by RIKEN and the like can be utilized.

nt ES cell is an ES cell derived from cloned embryo produced by nucleus transplantation technology, and has almost the same properties as ES cell derived from fertilized egg (Wakayama T. et al. (2001), Science, 292:740-743; S. Wakayama et al. (2005), Biol. Reprod., 72:932-936; Byrne J. et al. (2007), Nature, 450:497-502). That is, ES cell established from the inner cell mass of blastocyst derived from cloned embryo obtained by replacing the nucleus of unfertilized egg with the nucleus of somatic cell is nt ES (nuclear transfer ES) cell. For the production of nt ES cell, a combination of nucleus transplantation technology (Cibelli J.B. et al. (1998), Nature Biotechnol., 16:642-646) and ES cell producing technique (mentioned above) is used (Kiyoka Wakayama et al. (2008), experiment medicine, vol. 26, No. 5 (Extra edition), pages 47 - 52). In nuclear transplantation, the nucleus of somatic cell is injected into an enucleated unfertilized egg of a mammal and cultured for several hours to achieve reprogramming.

Pluripotency germ stem cell is a pluripotent stem cell derived from a germ stem cell (GS cell). Similar to ES cell, this cell can be induced to differentiate into cells of various lineages, and has properties such as being able to produce a chimeric mouse when transplanted into mouse blastocyst, and the like (Kanatsu-Shinohara M. et al. (2003) Biol. Reprod., 69:612-616; Shinohara K. et al. (2004), Cell, 119:1001-1012). It is self-renewable in a culture medium containing glial cell linederived neurotrophic factor (GDNF), and germ stem cells can be obtained by repeating passage under the same culture conditions as for ES cell ((2008), experiment medicine, vol. 26, No. 5 (Extra edition), pages 41 - 46, YODOSHA CO., LTD. (Tokyo, Japan)).

EG cell is a cell with pluripotency similar to that of ES cell, and is established from a primordial germ cell in the viviparous stage. It can be established by culturing a primordial germ cell in the presence of substances such as LIF, bFGF, stem cell factor and the like (Matsui Y. et al. (1992), Cell, 70:841-847; J.L. Resnick et al. (1992), Nature, 359:550-551) .

### -step of causing formation of cytotoxicity marker (step (1a))-

The complement to be used in the above-mentioned step (1a) is not particularly limited as long as it can form a cytotoxicity marker. Examples include complements contained in serum (e.g., human serum, bovine serum, horse serum, sheep serum, goat serum, pig serum, llama serum, dog serum, chicken serum, donkey serum, cat serum, rabbit serum, guinea pig serum, hamster serum, rat serum, mouse serum, etc.)), complements contained in the culture supernatants of liver and vascular endothelial cells (including organoids of these organs), each complement component (e.g., C1q, C1r, C1s, C2, C4, C3, C3a, C5s, C3b, C5a, C5b6789, combinations of these, etc.), and the like. In the following, a solution containing a complement such as the above-mentioned sera and culture supernatants are sometimes referred to as a "complement source". The present inventors have confirmed that the formation of cytotoxicity marker is promoted by combining plural types of complement sources as compared with a combination of each of them. Thus, it is preferable to combine plural types of complements or complement sources (e.g., a combination of serum and culture supernatant of organ organoid).

The complement or complement source to be used in this step may be prepared by a protein synthesis method known per se (e.g., solid phase synthesis process, liquid phase synthesis process, etc.), a method for isolating from a living organism, or a commercially available one may also be used. Alternatively, a complement can also be obtained by introducing a gene encoding complement into a host cell such as Escherichia coli, and the like to cause production of a protein. The gene encoding the complement can also be obtained by, preparing cDNA using genomic DNA extracted from a cell carrying the gene or from an extracted mRNA, and amplifying the nucleic acid of a desired length by the PCR method using the DNA as a template, or by chemical synthesis using a commercially available automatic DNA/RNA synthesizer or the like. The protein thus obtained can be purified and isolated by a known purification method, such as solvent extraction, distillation, column chromatography, liquid chromatography, recrystallization, combination of these, and the like.

The present inventors have previously confirmed that an artificially produced organoid secretes complement. Therefore, the culture supernatant of the aforementioned organoid can also be used as a complement source. Examples of such organoid include a three-dimensional structure produced only from vascular endothelial cells, a three-dimensional structure produced from vascular endothelial cells and hepatocytes (characterized by having a vascular network composed of vascular endothelial cells between hepatocytes) and the like. These organoids are generally produced by a culture method known per se (e.g., Nature Cell Biology 18, 246-254 (2016)). Specifically, as a method for producing an organoid, for example, the method described in Nahmias Y. et al., Tissue Eng., 12(6), 2006, pp. 1627-1638, and the like can be mentioned.

The vascular endothelial cells used for producing the aforementioned organoid may be hemogenic endothelial cells (HEC) or non-hemogenic endothelial cells (non-HEC). HEC is a vascular endothelial cell capable of producing hematopoietic stem cell (having hematopoietic ability), and is also called a blood cell-producing vascular endothelial cell. Only one of HEC and non-HEC may be used, both HEC and non-HEC may be used, progenitor cells thereof may be used, or any combination of HEC, non-HEC and progenitor cells thereof may also be used. Examples of the progenitor cells of the aforementioned vascular endothelial cells include cells present in the differentiation process from Flk-1 (CD309, KDR)-positive progenitor cells of vascular endothelial cells (e.g., lateral plate mesodermal cell) into HEC cells (see Cell Reports 2, 553-567, 2012).

Hepatocytes to be used for producing such organoids may be differentiated hepatocytes, or a cell whose differentiation fate into hepatocyte has been determined but has not yet differentiated into hepatocyte (undifferentiated hepatocyte), so-called liver progenitor cell (e.g., liver endodermal cell). Differentiated hepatocyte or undifferentiated hepatocyte may be a cell collected from a living body (isolated from the liver in the living body), or a cell obtained by differentiating a pluripotent stem cell such as ES cell, iPS cell and the like, a liver progenitor cell, or other cell having the ability to differentiate into hepatocyte. The cell that can differentiate into hepatocyte can be produced, for example, according to K.Si-Taiyeb, et al. Hepatology, 51 (1): 297- 305(2010), T. Touboul, et al. Hepatology. 51(5):1754-65. (2010).

In addition, mesenchymal stem cells may also be used in the production of the aforementioned organoid. Such mesenchymal stem cell may be a differentiated cell(differentiation mesenchymal cell), or a cell whose differentiation fate into mesenchymal cell has been determined but has not yet differentiated into mesenchymal cell (undifferentiated mesenchymal cell), so-called mesenchymal stem cell. What is referred to by the terms such as mesenchymal stem cell, mesenchymal progenitor cell, mesenchymal cell (R. Peters, et al. PLoS One. 30; 5(12):e15689.(2010)) and the like used by those skilled in the art corresponds to the "mesenchymal cell" in the present specification.

The ratio of the number of vascular endothelial cells and hepatocytes for producing the aforementioned organoid can be adjusted within an appropriate range such that, for example, the desired components will be contained in the collected culture supernatant. In one embodiment of the present invention, the ratio of the number of vascular endothelial cells to hepatocytes (vascular endothelial cells: hepatocytes) is typically 1:0.1 - 5, preferably 1:0.1 - 2. Even when mesenchymal stem cells are used, the ratio can be appropriately adjusted and, in a preferred embodiment, the ratio of the number of cells (vascular endothelial cells:hepatocytes: stem cells) to be used is 7:10:1.

When producing an organoid, it is preferable to use a medium for organoid in which a medium for vascular endothelial cells and a medium for hepatocytes (culture medium) are mixed at an appropriate ratio (e.g., 1:1)). Examples of the medium for vascular endothelial cells include DMEM/F-12 (Gibco), Stempro-34 SFM (Gibco), Essential 6 medium (Gibco), Essential 8 medium (Gibco), EGM (Lonza), BulletKit (Lonza), EGM-2 (Lonza), BulletKit (Lonza), EGM-2 MV (Lonza), VascuLife EnGS Comp Kit (LCT), Human Endothelial-SFM Basal Growth medium (Invitrogen), human microvascular endothelial cellular proliferation medium (TOYOBO) and the like. The medium for vascular endothelial cell may contain one or more kinds of additives selected from B27 Supplements (GIBCO), BMP4 (bone morphogenic factor 4), GSKβ inhibitor (e.g., CHIR99021), VEGF (vascular endothelial cell growth factor), FGF2 (Fibroblast Growth Factor (to be also referred to as bFGF (basic fibroblast growth factor))), Folskolin, SCF (Stem Cell Factor), TGFβ receptor inhibitor (e.g., SB431542), Flt-3L (Fms-related tyrosine kinase 3 ligand), IL-3 (Interleukin 3), IL-6 (Interleukin 6), TPO (thrombopoietin), hEGF (recombinant human epithelial cell growth factor), hydrocortisone, ascorbic acid, IGF1, FBS (bovine fetal serum), antibiotic (e.g., gentamicin, amphotericin B), heparin, L-glutamine, phenol red, BBE and the like. The amount of these additives to be added can be determined as appropriate by those of ordinary skill in the art, by reference to general culture conditions for culturing vascular endothelial cells.

Examples of the medium for hepatocytes include RPMI (Fujifilm Corporation), HCM (Lonza) and the like. The medium for hepatocytes may contain one or more kinds of additives selected from Wnt3a, activin A, BMP4, FGF2, FBS (bovine fetal serum), HGF (hepatocyte growth factor), oncostatin M (OSM), dexamethasone (Dex), and the like. The amount of these additives to be added can be determined as appropriate by those of ordinary skill in the art, by reference to general culture conditions for culturing hepatocytes. Alternatively, as the medium for hepatocytes, a medium for hepatocytes containing at least one kind selected from ascorbic acid, BSA-FAF, insulin, hydrocortisone and GA-1000, HCM BulletKit (Lonza) after removal of hEGF (recombinant human epithelial cell growth factor), a medium obtained by adding 1% B27 Supplements (GIBCO) and 10 ng/mL hHGF (Sigma-Aldrich) to RPMI1640 (Sigma-Aldrich), a medium obtained by removing hEGF (recombinant human epithelial cell growth factor) from GM BulletKit (Lonza) and HCM BulletKit (Lonza), mixing them at 1:1, and adding dexamethasone, oncostatin M and HGF and the like may also be used.

The culture supernatant of organoid can be obtained by collecting the medium supernatant in which the organoid produced by the aforementioned method is cultivated. The collected culture supernatant may be concentrated as necessary.

The method of adding a complement to cells produced from stem cells or the method of contacting the cells with the complement in step (1a) is not particularly limited. For example, the complement or complement source is added to the medium in which the cells are cultured, or the cells are transferred to or seeded in a medium added with the complement or complement source in advance, or the like.

The formation of a cytotoxicity marker is not particularly limited. For example, it can be performed by culturing cells prepared from stem cells in a medium containing the cells and a complement. Examples of the medium generally used in the aforementioned culture include BME medium, BGJb medium, CMRL 1066 medium, Glasgow MEM medium, Improved MEM (IMEM) medium, Improved MDM (IMDM) medium, medium 199 medium, Eagle MEM medium, α MEM medium, DMEM medium (High glucose, Low glucose), DMEM/F12 medium, Ham's medium, RPMI 1640 medium, Fischer's medium, a mixed medium of these, and the like. In addition, the aforementioned medium for vascular endothelial cell, medium for hepatocytes, medium for organoid, or a mixed medium of these may also be used.

In addition to the above-mentioned components, amino acid, L-glutamine, GlutaMAX (product name), non-essential amino acid, vitamin, antibiotic (e.g., Antibiotic-Antimycotic (sometimes to be referred to as AA in the present specification), penicillin, streptomycin, or a mixture of these), antibacterial agent (e.g., amphotericin B), antioxidant, pyruvic acid, buffering agent, inorganic salts and the like may also be added as necessary to the aforementioned medium.

The culture period is not particularly limited as long as a cytotoxicity marker can be formed. For example, 0.5 hr - 96 hr is preferable, 4 hr - 48 hr is more preferable. The culture temperature is not particularly limited and it is preferably 30 - 40°C (e.g., 37°C). The concentration of carbon dioxide in the culture container is, for example, about 5%.

Detection or measurement of a decrease in the amount of cytotoxicity marker is not particularly limited, and can be performed by immunological assay using antibody (e.g., anti-MAC, C9 antibody that can detect MAC, anti-ATP antibody, anti-Caspase 3,7 antibody, anti-CD59 antibody, anti-LDH antibody, etc.), ELISA, immunostaining, Western blotting, and flow cytometry. To quantify the expression level based on the results of immunostaining, Western blotting, and the like, the obtained image data can also be quantified using image processing software (e.g., ImageJ, etc.). When the cytotoxic marker has enzymatic activity such as LDH, the cytotoxicity marker can also be detected or measured using the enzyme activity as an index and using a kit as necessary (e.g., LDH Cytotoxicity Assay Kit (funakoshi), etc.) and the like.

When serum is used as the complement source, the concentration of the serum in the medium is preferably 1%(v/v) - 50%(v/v) is preferable, more preferably 3%(v/v) - 20%(v/v). When culture supernatant of organ organoid is used as the complement source, the concentration of the culture supernatant in the medium is preferably 1%(v/v) - 50%(v/v), more preferably 3%(v/v) - 20%(v/v). The concentration can be determined as appropriate by those of ordinary skill in the art, by reference to the kind of the complement to be used, form of supply and the like.

In step (1a), moreover, a complement may be brought into contact with a cell produced from a stem cell by further adding a complement activation factor to the medium in order to promote the formation of a cytotoxic marker. For example, a complement activation factor is added to the medium in which a cell produced from a stem cell is cultured, or the cell is transferred to or seeded in a medium added with a complement activation factor in advance, or the like. The complement activation factor may be added to a cell produced from a stem cell, before or after addition of a complement or a complement source. Such complement activation factor is not particularly limited as long as it can promote the formation of a cytotoxicity marker, and examples thereof include LPS (Lipopolysaccharide), zymosan which is a polysaccharide in the cell wall of yeast, and the like.

-a step of selecting a therapeutic drug candidate that decreases the amount of the cytotoxicity marker (step (2a))-Examples of the therapeutic drug candidate to be used in step (2a) include cell extract, cell culture supernatant, microorganism fermentation product, extract derived from marine organism, plant extract, purified protein or crude protein, peptide, non-peptide compound, synthesized low-molecular-weight compound, and natural compound.

The therapeutic drug candidate to be used in step (2a) can be obtained using any of many approaches in the combinatorial library method known in the technical field including (1) biological library method, (2) synthetic library method using deconvolution, (3) "one-bead one-compound" library method, and (4) synthetic library method using affinity chromatography selection. While a biological library method using affinity chromatography selection is limited to peptide library, other 4 approaches are applicable to peptide, non-peptide oligomer, and low-molecular-weight compound library of the compound (Lam (1997) Anticancer Drug Des. 12: 145-67). Examples of the synthesis method of molecule library can be found in the technical field (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). Compound library can be produced as a solution (refer to Houghten (1992) Bio/Techniques 13: 412-21) or bead (Lam (1991) Nature 354: 82-4), chip (Fodor (1993) Nature 364: 555-6), bacterium (US Patent No. 5,223,409), spore (US Patent Nos. 5,571,698, 5,403,484, and 5,223,409), plasmid (Cull et al. (1992) Proc. Natl. Acad. Sci. USA 89: 1865-9) or phage (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; US-B-2002103360).

A method for adding a therapeutic candidate substance, or a method for bringing a cell produced from a stem cell into contact with a therapeutic candidate substance in step (2a) is not particularly limited. For example, a therapeutic candidate substance is added to a medium containing the cells after step (1a), or the cell is transferred to or seeded in a medium added with a therapeutic candidate substance in advance, or the like. In step (2a), a therapeutic candidate substance that decreases the amount of a cytotoxicity marker can be selected as a therapeutic agent for diseases involving excessive activation of a complement, or can be determined to be the therapeutic agent.

Alternatively, the therapeutic candidate substance is added in advance to the medium before step (1a), and step (1a) can be performed using the medium. In this case, for example, when the amount of the cytotoxicity marker formed is lower than that of a control to which a therapeutic candidate substance is not added or a control to which a substance having no therapeutic effect is added, the therapeutic candidate substance can be selected as a therapeutic agent for diseases involving excessive activation of a complement, or can be determined to be the therapeutic agent.

When the screening method of the present invention is performed, a positive control such as a substance having a therapeutic effect on a disease involving excessive activation of a complement (e.g., Eculizumab etc.) and the like and/or a negative control such as a placebo and the like may also be used.

### -2. A method for evaluating cytotoxicity caused by excessive activation of complement-

The present invention provides a method for evaluating cytotoxicity of a test substance caused by excessive activation of a complement, using a cytotoxicity marker as an index (hereinafter sometimes to be referred to as "the evaluation method of the present invention"). The evaluation method of the present invention includes
(1b) a step of adding a test substance to a cell produced from a stem cell,
(2b) a step of measuring the production amount of the cytotoxicity marker, and
(3b) a step of evaluating cytotoxicity caused by excessive activation of the complement from the production amount of the cytotoxicity marker.

As shown in the Examples described below, the number of cells significantly decreased and the amount of MAC formation increased with human serum when Oxaliplatin known to cause hepatic sinusoidal endothelial cell injury was administered to human hepatic sinusoidal endothelial cells. That is, it was shown that the cytotoxicity of Oxaliplatin can be evaluated based on the amount of MAC formation. Therefore, according to the evaluation method of the present invention, it is possible to evaluate or predict whether or not the test substance has cytotoxicity due to complement activation. Therefore, according to the evaluation method of the present invention, it is possible to evaluate or predict whether or not a test substance has cytotoxicity caused by the activation of the complement. When the test substance is a therapeutic drug or a therapeutic drug candidate for any disease, it is possible to evaluate or predict, using a cytotoxicity marker as an index, whether or not the substance induces activation of a complement, namely, whether it has toxicity through activation of a complement (side effect). Examples of such test substance include cell extract, cell culture supernatant, microorganism fermentation product, extract derived from marine organism, plant extract, purified protein or crude protein, peptide, non-peptide compound, synthesized low-molecular-weight compound, and natural compound.

The aforementioned test substance can also be obtained using any of many approaches in the combinatorial library method known in the technical field including (1) biological library method, (2) synthetic library method using deconvolution, (3) "one-bead one-compound" library method, and (4) synthetic library method using affinity chromatography selection. While a biological library method using affinity chromatography selection is limited to peptide library, other 4 approaches are applicable to peptide, non-peptide oligomer, and low-molecular-weight compound library of the compound (Lam (1997) Anticancer Drug Des. 12: 145-67). Examples of the synthesis method of molecule library can be found in the technical field (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). Compound library can be produced as a solution (refer to Houghten (1992) Bio/Techniques 13: 412-21) or bead (Lam (1991) Nature 354: 82-4), chip (Fodor (1993) Nature 364: 555-6), bacterium (US Patent No. 5,223,409), spore (US Patent Nos. 5,571,698, 5,403,484, and 5,223,409), plasmid (Cull et al. (1992) Proc. Natl. Acad. Sci. USA 89: 1865-9) or phage (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; US-B-2002103360).

The definition and specific examples of the cytotoxic marker are as described in the above-mentioned 1. The cell produced from a stem cell is exemplified by those described in the above-mentioned 1.

A method for adding a therapeutic candidate substance to a cell produced from a stem cell or a method for bringing of the cell into contact with a therapeutic candidate substance in step (1b) is not particularly limited. For example, a therapeutic candidate substance is added to a medium where the cells are cultivated, or the cell is transferred to or seeded in a medium added with a therapeutic candidate substance in advance, or the like.

The amount of cytotoxic marker produced in step (2b) can be measured by the same method as the method described in step (2a) of the above-mentioned 1.

In step (3b), for example, when the amount of the cytotoxicity marker formed increases as compared with that of a control to which a test substance is not added, a control to which a substance incapable of causing excessive activation of a complement is added, or a cell before addition of a test substance, the test substance can be evaluated to have cytotoxicity caused by excessive activation of the complement. When the amount of the cytotoxicity marker formed does not change or decreases, the test substance can be evaluated to have no cytotoxicity caused by excessive activation of the complement.

### -3. Screening kit for a therapeutic drug for a disease involving excessive activation of a complement -

Also, the present invention provides a kit for screening for a therapeutic drug for a disease involving excessive activation of a complement (hereinafter sometimes to be referred to as "the screening kit of the present invention"). The screening method of the present invention may be performed using the screening kit of the present invention. The screening kit of the present invention contains
(i-a) a cell produced from a stem cell,
(ii-a) a complement or a complement source, and
(iii-a) a reagent that detects a cytotoxicity marker.

The disease involving excessive activation of a complement is exemplified by those described in the above-mentioned 1. The cell produced from a stem cell in the aforementioned (i-a) is exemplified by those described in the above-mentioned 1. The complement or complement source in the aforementioned (ii-a) is also exemplified by those described in the above-mentioned 1. Examples of the reagent in the aforementioned (iii-a) include an antibody recognizing a cytotoxicity marker (e.g., anti-MAC, C9 antibody that can detect MAC, anti-ATP antibody, anti-Caspase 3,7 antibody, CD59 antibody, anti-LDH antibody etc.) and the like. When the aforementioned reagent contains two or more kinds of antibodies, the reagent can be provided as a reagent kit containing each antibody in a separate reagent. The antibody contained in the reagent of the present invention may be provided, for example, in a form bound to a fluorescent dye, a metal isotope or a bead (e.g., magnetic bead).

The screening kit of the present invention may contain, in addition to the above-mentioned (i-a) - (iii-a), other substances used for screening for a therapeutic drug. These other substances may be provided in coexistence with the above-mentioned (i-a) - (iii-a), or may be provided together with a separate reagent, as long as they do not adversely affect the reaction. Examples of the aforementioned other substance include the complement activation factor described in the above-mentioned 1., the therapeutic drug candidate described in the above-mentioned 1., the medium described in the above-mentioned 1., the positive control and/or negative control described in the above-mentioned 1., reaction buffer, competitor antibody, labeled secondary antibody (e.g., mouse anti-rabbit IgG labeled with peroxidase, alkaline phosphatase, etc., when the primary antibody is a rabbit antibody), blocking solution, ELISA plate and the like. In addition, the screening kit of the present invention may contain an instruction manual describing how to use the kit and the reagent.

### -4. Kit for evaluating cytotoxicity caused by excessive activation of complement -

In addition, the present invention provides a kit for evaluating cytotoxicity of a test substance caused by excessive activation of a complement (hereinafter sometimes to be referred to as "the evaluation kit of the present invention"). The evaluation method of the present invention may be performed using the evaluation kit of the present invention. The evaluation kit of the present invention contains
(i-b) a cell produced from a stem cell, and
(ii-b) a reagent that detects a cytotoxicity marker associated with the complement.

The cell produced from a stem cell in the aforementioned (i-b) is exemplified by those described in the above-mentioned 1. The reagent of the aforementioned (ii-b) is exemplified by those described in the above-mentioned 3. (iii-a). When the aforementioned reagent contains two or more kinds of antibodies, the reagent can be provided as a reagent kit containing each antibody in a separate reagent. The present invention reagent antibody may be provided, for example, in a form bound to a fluorescent dye, a metal isotope or a bead (e.g., magnetic bead) .

The evaluation kit of the present invention may contain, in addition to the above-mentioned (i-b) and (ii-b), other substances used for evaluating cytotoxicity. These other substances may be provided in coexistence with the above-mentioned (i-b) and (ii-b), or may be provided together with a separate reagent, as long as they do not adversely affect the reaction. Examples of the aforementioned other substance include the test substance described in the above-mentioned 2., the medium described in the above-mentioned 1., the positive control and/or negative control described in the above-mentioned 1., reaction buffer, competitor antibody, labeled secondary antibody (e.g., mouse anti-rabbit IgG labeled with peroxidase, alkaline phosphatase, etc., when the primary antibody is a rabbit antibody), blocking solution, ELISA plate and the like. In addition, the evaluation kit of the present invention may contain an instruction manual describing how to use the kit and the reagent, evaluation criteria and the like.

The present invention is explained further specifically in the following Examples; however, the present invention is not limited thereby.

### [Example]

In the following Examples, the cell marker was confirmed by flow cytometry, immunostaining, and/or quantitatively PCR.

### (confirmation of CD31, CD73 and CD144 markers)

The CD31, CD73 and CD144 markers were confirmed by reacting the cells with fluorescence-labeled anti-CD31 antibody (FITC Mouse anti-Human CD31, BD Pharmingen), anti-CD73 antibody (CD73-PE, human, Miltenyi Biotec or CD73-APC, human, Miltenyi Biotec), and anti-CD144 antibody (PE Mouse anti-Human CD144, BD Pharmingen), followed by flow cytometry (FACS Fortessa (BD)). As a marker negative sample, undifferentiated human iPS cells (1383D2; Center for iPS Cell Research and Application, Kyoto University) were used. In addition, a negative control was prepared by reacting an antibody that does not recognize the target marker and has the same isotype as the labeled antibody.

### (confirmation of HNF4α marker)

HNF4α marker was confirmed by immunostaining and quantitatively PCR.

### (Immunostaining method)

The cells were fixed with 4% para-formaldehyde (PFA) in PBS at room temperature for 15 min, blocked with donkey and goat sera, immunostained with an anti-HNF4α antibody (santacruz) and a fluorescence-labeled secondary antibody (Novex Donkey anti-Goat IgG (H+L) Secondary Antibody (invitrogen)) that binds to the anti-HNF4α antibody, and then confirmed with a fluorescence microscope.

### (Quantitative PCR)

Using PureLink RNA Mini Kit (invitrogen), RNA was extracted from the cells, and cDNA was synthesized using High-Capacity cDNA Reverse Transcription Kit (invitrogen). Using THUNDERBIRD Probe qPCR Mix (TOYOBO) and QuantStudio 7Flex realtime PCR system (Applied Biosystems), quantitative PCR was performed. The PCR primers (Fw: forward primer; Rv: reverse primer) and probes used were as follows: HNF4α
Fw 5'-TCAGACCCTGAGCCACCT-3' (SEQ ID NO: 1)
Rv 5'-AGCAACGGACAGATGTGTGA-3' (SEQ ID NO: 2)
probe; Universal ProbeLibrary Probe 027 (Roche) AFP
Fw 5'-TCCTTGTAAGTGGCTTCTTGAAC-3' (SEQ ID NO: 3)
Rv 5'-TGTACTGCAGAGATAAGTTTAGCTGAC-3' (SEQ ID NO: 4)
probe; Universal ProbeLibrary Probe 061 (Roche) ALB
Fw 5'-CTTCCCTTCATCCCGAAGTT-3' (SEQ ID NO: 5)
Rv 5'-AATGTTGCCAAGCTGCTGA-3' (SEQ ID NO: 6)
probe; Universal ProbeLibrary Probe 027 (Roche) 18s rRNA (Endogenous Control)
   EUK 18s rRNA (20×) (ABI)

### [Example 1]

Detection of membrane attack complex in iPS cell-derived human vascular endothelial cells by using iPS cell-derived liver organoid culture supernatant and human serum

### (1-1) Production of iPS human non-hemogenic endothelial cell

Human iPS cells (1383D2; Center for iPS Cell Research and Application, Kyoto University) were cultured in DMEM/F-12 (Gibco) (10 ml) supplemented with 1% B-27 Supplements (GIBCO), BMP4 (25 ng/ml) , CHIR99021 (8 µM) at 5% CO₂, 37°C for 3 days to induce mesodermal cells. The cells were further cultured in Stempro-34 SFM (Gibco) (10 ml) supplemented with VEGF (200 ng/ml) , Folskolin (2 µM) at 5% CO₂, 37°C for 7 days to give a CD31-positive, CD73-positive and CD144-positive human non-hemogenic endothelial cell population.

### (1-2) Production of human hepatic endoderm (HE)

Human iPS cells (1383D2; Center for iPS Cell Research and Application, Kyoto University) were cultured in RPMI (FUJIFILM) (2 ml) supplemented with Wnt3a (50 ng/mL), activin A (100 ng/ml) at 5% CO₂, 37°C for 5 days to induce endodermal cells. The obtained endodermal cells were further cultured in the same medium supplemented with 1% B27 Supplements (GIBCO), FGF2 (10 ng/ml) at 5% CO₂, 37°C for 5 days to give an AFP-, ALB- and HNF4α- positive human liver endodermal cell population.

### (1-3) Production of human mesenchymal stem cell (MC)

Human iPS cells (1383D2; Center for iPS Cell Research and Application, Kyoto University) were cultured in DMEM/F-12 (Gibco) (10 ml) supplemented with 1% B-27 Supplements (GIBCO), BMP4 (25 ng/ml) , CHIR99021 (8 µM) at 5% CO₂, 37°C for 3 days to induce mesodermal cells. The cells were further cultured with PDGFBB (10 ng/ml), activin A (2 ng/ml) at 5% CO₂, 37°C for 3 days. Then the cells were further cultured in DMEM/F-12 (Gibco) (10 ml) supplemented with 1% B-27 Supplements (GIBCO), FGF (10 ng/ml), BMP4 (12 ng/ml) at 5% CO₂, 37°C for 3 days to give CD31-negative, CD73-positive human mesenchymal stem cells.

### (1-4) Production of organoid (three-dimensional structure)

The produced human hepatic endoderm (HE), human non-hemogenic endothelial cell (non-HEC) and human mesenchymal stem cell (MC) were mixed at the cell number ratio of 10:7:1 (total number 18×10⁶ cells), and cocultured on a three-dimensional culture container Elplasia (KURARAY CO. LTD.) for one day at 5% CO₂, 37°C to produce aggregates. The culture medium used for this coculture was a mixture of hepatocyte medium (A) which is HCM (Lonza) supplemented with FBS (5%), HGF (10 ng/ml), OSM (20 ng/ml), Dex (100 nM), and vascular endothelial cell medium (A) which is Stempro-34 SFM (Gibco) supplemented with VEGF (50 ng/ml), FGF2 (10 ng/ml) at a volume ratio of 1:1 (to be referred to as "organoid medium (A)" in the present specification). Thereafter, the medium was replaced with this medium every day, and the culture supernatant on day 14 was collected and used as a complement component. The present inventors confirmed that formation of MAC is observed by using human serum and any of the aforementioned culture supernatants as a complement component, and that the MAC formation effect is improved by using the complement source in combination as compared to when it is used alone. Therefore, in the following Examples, human serum and the aforementioned culture supernatant were used as the complement source.

### (1-5) Detection of membrane attack complex in iPS cell-derived human non-hemogenic endothelial cell

iPS cells (1383D2; Center for iPS Cell Research and Application, Kyoto University) (4000 cells) were seeded on a 96-well plate (corning), and iPS-derived human non-hemogenic endothelial cells were produced on the plate according to the method in the above-mentioned 1-1. The medium for the cells containing a CD59 antibody (1 µg/ml) was added at 100 µl/96 well, and the cells were incubated at 37°C for 1 hr. Then, an equal amount of the same medium (containing 6%(v/v) human serum [BIOPREDIC Inc. SER018A050F018], 20% (v/v) culture supernatant collected in (1-4), LPS (Lipopolysaccharide) 2 µg/ml) was added to the cell culture medium (therefore, the concentration of human serum in the medium after addition was 3%(v/v), and the concentration of the culture supernatant collected in (1-4) was 10%(v/v)), and the cells were incubated at 37°C for 24 hr. The next day, the medium was removed, the cells were washed once with PBS(-) (GIBCO), 4% para-formaldehyde was added to the cells at 50 µl/96 well and the cells were left standing for 15 min at room temperature. Then, the cells were washed 3 times with PBS(-), and preserved at 4°C overnight. To the cells was added PROTEIN BLOCK SERUM-FREE blocking solution [DAKO, X909] at 50 µl/96 well and the cells were left standing for 60 min at room temperature for blocking. Thereafter, a primary antibody solution (1/200 Anti-terminal complement complex, HCB Hycult Biotech, HM2167 with 1% blocking solution in PBS(-)) was added at 50 µl/96 well, and the cells were left standing for 60 min at room temperature or at 4°C overnight. After washing 3 times with PBS(-), a secondary antibody solution (1/1000, Alexa Flour 555, 1/1000 DAPI-HCB Hycult Biotech, HM2167 with 1% blocking solution in PBS(-)) was added at 50 µl/96 well, and the cells were incubated at 37°C for 60 min. After washing 3 times with PBS(-), and quantitative analysis was performed by a cell analyzer 6500HS (GE Healthcare). The obtained numerical value was calculated as a value relative to the value of 4',6-diamidino-2-phenylindole (DAPI). As a negative control, an inactivated serum was used. The results are shown in Fig. 1.

### [Example 2]

The action of complement factor V C5 antibody Eculizumab (AB00296, Absolute Antibody Ltd) on the formation of complement membrane attack complex in iPS cell-derived non-hematopoietic human vascular endothelial cells by using iPS cell-derived liver organoid culture supernatant and human serum was tested. The membrane complex was formed and detected by the method described in the above-mentioned (1-5) except that Eculizumab was added at a concentration of 2.028 - 676 nano mol/L simultaneously with the serum. The results are shown in Fig. 2.

### [Example 3]

### Detection of membrane attack complex in iPS cell-derived human nerve cell

### (3-1) Production of iPS cell-derived human nerve cell

Human iPS cells (253G1; Center for iPS Cell Research and Application, Kyoto University) were induced to differentiate into iPS cell-derived human neural stem cell and nerve cell according to Yan Y et al., Stem Cells Trans Med, 2:862-870. (2013) and using PSC Neural Induction medium (Thermo Fisher Scientific).

### (3-2) Detection of membrane attack complex in iPS cell-derived human nerve cell

Differentiation was induced on a 96-well plate for 15 days according to the method of the above-mentioned 3-1 to produce iPS cell-derived human nerve cells. The membrane attack complex associated with complement activation was detected according to the method of the above-mentioned (1-5). The results are shown in Fig. 3 (upper Figure). The formation of MAC was observed by using human serum.

### [Example 4]

### Detection of membrane attack complex in iPS cell-derived human hepatocyte

### (4-1) Production of iPS cell-derived human hepatocyte

According to the method of the above-mentioned 1-2, human iPS cells (1383D2; Center for iPS Cell Research and Application, Kyoto University) were induced to differentiate into an AFP-, ALB- and HNF4α-positive human liver endodermal cell population 10 days later. Furthermore, the cells were cultured in HCM medium (LONZA) supplemented with 5% FBS, HGF (10 ng/mL), OSM (20 ng/ml), Dex (100 nM) at 5% CO₂, 37°C for 11 days to induce iPS cell-derived human liver progenitor cells.

### (4-2) Detection of membrane attack complex in iPS cell-derived human hepatocyte

Differentiation was induced on a 96-well plate for 21 days according to the method of the above-mentioned 3-1 to produce iPS cell-derived human hepatocytes. The membrane attack complex associated with complement activation was detected according to the method of the above-mentioned (1-5). The results are shown in Fig. 3 (middle Figure). The formation of MAC was observed by using human serum.

### [Example 5]

### Detection of membrane attack complex in iPS cell-derived human retinal pigment epithelial cell

### (5-1) Production of iPS cell-derived human retinal pigment epithelial cell

Human iPS cells (253G1; Center for iPS Cell Research and Application, Kyoto University) were induced to differentiate into iPS cell-derived human retinal pigment epithelial cells according to the production method of WO 2015053375 A1.

### (5-2) Detection of membrane attack complex in iPS cell-derived human retinal pigment epithelial cell

Differentiation was induced on a 96-well plate for 21 days according to the method of the above-mentioned 3-1 to produce iPS cell-derived human retinal pigment epithelial cells. The membrane attack complex associated with complement activation was detected according to the method of the above-mentioned (1-5). The results are shown in Fig. 3 (lower Figure). The formation of MAC was observed by using human serum.

### [Example 6]

### Detection of membrane attack complex in iPS cell-derived human hepatic sinusoidal endothelial cell with Oxaliplatin administration

### (6-1) Production of iPS cell-derived human hepatic sinusoidal endothelial cell

Human iPS cells (625A4; Center for iPS Cell Research and Application, Kyoto University) were cultured in AK02N (Ajinomoto Co., Inc.) (8 ml) at 5% CO₂, 37°C for 6 - 7 days to form an iPS cell colony with a diameter of 500-700 µm. The obtained colony was cultured in Essential 8 medium (Gibco) (8 ml) supplemented with BMP4 (80 ng/ml), VEGF (80 ng/ml) and CHIR99021 (2 µM) at 5% CO₂, 37°C for 2 days. Then the medium was replaced with Essential 6 medium (Gibco) (8 ml) supplemented with VEGF (80 ng/ml), FGF2 (25 ng/ml), SCF (50 ng/ml) and SB431542 (2 µM), and the cells were cultured at 5% CO₂, 37°C for 2 more days to induce lateral plate mesodermal cells. Thereafter, the medium was replaced with Stempro-34 SFM (Gibco)(8 ml) supplemented with VEGF (80 ng/ml), SCF (50 ng/ml), Flt-3L (50 ng/ml), IL-3 (50 ng/ml), IL-6 (50 ng/ml) and TPO (5 ng/ml), and the cells were cultured at 5% CO₂, 37°C for 2 days. The medium was replaced with a medium with the above-mentioned composition and without containing VEGF, and the cells were cultured at 5% CO₂, 37°C for 1 day to induce CD34-positive, CD73-negative hematopoietic vascular endothelial cells. The hematopoietic vascular endothelial cells were dissociated with TrpLE Express (Gibco), and reseeded on a 96-well plate thinly coated with Matrigel (BD Pharmingen) diluted 50-fold with PBS, cultured in Stempro-34 SFM (Gibco) (150 µl) supplemented with Rock inhibitor (10 µM), VEGF (10 ng/ml), SCF (50 ng/ml), Flt-3L (10 ng/ml), IL-3 (10 ng/ml), IL-6 (10 ng/ml), TPO (10 ng/ml), IL-11 (5 ng/ml), IGF-1 (25 ng/ml) and EPO (2 Unit/mL) for 24 hr. The medium was replaced with a medium with the above-mentioned composition and without containing the Rock inhibitor, and the cells were cultured at 5% CO₂, 37°C for 2 days to give CD34-positive, CD32-positive, Factor VIII-positive hepatic sinusoidal endothelial cells.

### (6-2) Detection of membrane attack complex in iPS cell-derived human hepatic sinusoidal endothelial cell

According to the method of the above-mentioned 6-1, iPS cell-derived human hepatic sinusoidal endothelial cells were produced on a 96-well plate. The medium for the cells containing a CD59 antibody (1 µg/ml) was added at 100 µl/96 well, and the cells were incubated at 37°C for 1 hr. Then, an equal amount of the same medium (containing 6%(v/v) human serum [BIOPREDIC Inc. SER018A050F018], 20% (v/v) culture supernatant collected in (1-4), and 2 mM Oxaliplatin (final concentration 20 µM) or the same solvent (ethanol)) was added the cell culture medium (therefore, the concentration of human serum in the medium after addition was 3%(v/v), and the concentration of the culture supernatant collected in (1-4) was 10%(v/v)), and the cells were incubated at 37°C for 24 hr. Thereafter, the membrane attack complex associated with complement activation was detected according to the method of the above-mentioned (1-5), and the number of cells was quantified with DAPI as an index. The results are shown in Fig. 4 (upper Figure). Due to Oxaliplatin, a significant decrease in the number of cells was observed (Fig. 4A) and the amount of MAC formed by human serum significantly increased (Fig. 4B).

### [Industrial Applicability]

In the present invention, a therapeutic drug for a disease involving activation of a complement can be screened for while considering blood vessel injury that occurs in vivo due to the activation of the complement, by using a cytotoxicity marker associated with the complement as an index. Therefore, highly reliable drug discovery screening can be performed.

This application is based on a patent application No. 2019-066625 filed in Japan (filing date: March 29, 2019), the contents of which are incorporated in full herein.

## Claims

1. A method for screening for a therapeutic drug for a disease involving excessive activation of a complement, using a cytotoxicity marker associated with the complement as an index, comprising
(1a) a step of adding a complement to a cell produced from a stem cell to cause the cell to form a cytotoxicity marker associated with the complement, and
(2a) a step of adding a therapeutic drug candidate, and selecting a substance that decreases the amount of the cytotoxicity marker.

2. The method according to claim 1, further comprising adding a complement activation factor in step (1a).

3. A method for evaluating cytotoxicity of a test substance caused by excessive activation of a complement, using a cytotoxicity marker associated with the complement as an index, comprising
(1b) a step of adding a test substance to a cell produced from a stem cell,
(2b) a step of measuring the production amount of the cytotoxicity marker associated with the complement, and
(3b) a step of evaluating cytotoxicity caused by excessive activation of the complement from the production amount of the cytotoxicity marker.

4. The method according to claim 1 or 3, wherein the cell produced from a stem cell is a vascular endothelial cell, a hepatic sinusoidal endothelial cell, a nerve cell, an oligodendrocyte, a hepatocyte or a retinal pigment epithelial cell.

5. A kit for screening for a therapeutic drug for a disease involving excessive activation of a complement, comprising
(i-a) a cell produced from a stem cell,
(ii-a) a complement or a complement source, and
(iii-a) a reagent that detects a cytotoxicity marker associated with the complement.

6. A kit for evaluating cytotoxicity caused by excessive activation of a complement
(i-b) a cell produced from a stem cell, and
(ii-b) a reagent that detects a cytotoxicity marker associated with the complement.
